# EUROPEAN PATENT APPLICATION

(11) **EP 2 468 855 A1**
(43) Date of publication of application: **27.06.2012**
(21) Application number: 10809953.2
(22) Date of filing: 17.08.2010
(51) Int. Cl.: C12N 9/24, C07H 3/02, C12N 1/14, C12P 19/14

(54) **-GLUCANASE AND XYLANASE PREPARATION METHOD USING WHEAT BRAN, AND LIQUID CULTURE MEDIUM**

(30) Priority: 21.08.2009 JP 2009192216
(71) Applicant: Asahi Group Holdings, Ltd., Tokyo 130-8602 (JP)
(72) Inventor: FUKUDA, Kazuro, Moriya-shi Ibaraki 302-0106 (JP)
(74) Representative: Müller, Christian Stefan Gerd
(86) International application number: PCT/JP2010/063839
(87) International publication number: WO 2011/021612

(57) **Abstract**

To produce cellulase having excellent ability to decompose cellulosic resources containing xylan at low cost.

A method for producing β-glucanase and xylanase, comprising the step of culturing a microorganism classified under the genus Trichoderma by using a liquid culture medium which contains (a) wheat bran as a carbon source and (b) ammonia nitrogen or amino nitrogen as a nitrogen source.

## Description

### TECHNICAL FIELD

The present invention relates to a method for simultaneously and highly producing β-glucanase and xylanase, and a liquid culture medium useful for producing the enzymes.

### BACKGROUND ART

In order to effectively utilize cellulosic resources, a method for efficiently decomposing cellulose has been explored in recent years. Cellulose is mainly decomposed by microorganisms in nature, and it is known that various microorganisms such as bacteria, filamentous fungi and the like produce cellulolytic enzymes.

These microorganisms secrete the cellulolytic enzymes outside those bodies, and cellulose is decomposed by its action into glucose via mainly cello-oligosaccharide and cellobiose. Cellulolytic enzymes are generally called cellulase.

When cellulase is intended to be artificially produced, the genus Trichoderma is known as microorganisms secreting cellulase, and is widely utilized. Moreover, a method for secreting cellulase by culturing the microorganisms classified under the genus Trichoderma by using a culture medium containing nutrients such as a carbon source, a nitrogen source and the like is also known.

However, in the conventional method for producing cellulase, materials usable as a carbon source and pretreatment methods are limited. For example, crystalline cellulose is expensive, and even if inexpensive cellulosic resources are used, they generally require pretreatment such as heat treatment, alkali treatment and the like, that causes relatively high cost.

Patent Literature 1, for example, discloses a substrate for producing cellulase, which substrate is obtained by boiling used paper in a ferrous sulfate solution and can be inoculated with cellulase-producing microorganisms. In addition, Patent Literature 2 discloses a method for producing a substrate for producing cellulase, which substrate can be inoculated with Trichoderma reesei which is a cellulase-producing microorganism, by boiling pulverized bagasse with caustic alkali and treating with a hypochlorite solution.

In addition, the cellulase obtained by these conventional methods mainly contains β-glucanase, and has low xylanase activity and little ability to decompose cellulosic resources containing xylan, such as bagasse, rice straw and the like. Therefore, it is less effective for the purpose of effectively utilizing a variety of naturally-occurring cellulosic resources.

Patent Literature 3 discloses a method for producing cellulase, which method includes the step of collecting cellulase obtained by liquid culture of mutants of Trichoderma reesei. As carbon sources of media, a variety of materials with different chemical structures and characters such as cellulose powder, cellobiose, filter paper, general paper, sawdust, bran, chaff, bagasse, soymeal, coffee grounds and the like are listed (paragraph 0011).

Among these, however, only cellobiose (Example 1) and avicel (Example 2) are actually used for culturing operations, and production of cellulase has not been confirmed in the other materials, i.e. natural cellulosic materials.

Patent Literature 4 discloses a method for producing xylanase by culturing a microorganism classified under the genus Trichoderma by using a diluted alcohol distillation waste fluid of rye subjected to preliminary treatment such as removal of solid constituents, concentration of nonvolatile components, autoclave treatment of the concentrate and the like.

However, rye used as a carbon source in this technique is not easily available, and more complicated pretreatment is required. Thus, the technique causes high cost. In addition, the production amount of β-glucanase is rather decreased by this method.

Wheat bran is wheat skin (husks) left after milling wheat. Wheat bran contains a large amount of dietary fiber, and is used as food such as cereal and the like, as well as for solid media for culturing Aspergillus in brewing sake.

Wheat bran is also utilized as a material for liquid culture media, which are used for culturing microorganisms for the purpose of producing enzymes. Patent Literature 5, for example, describes the utilization of agriculture waste, plant fiber or the like such as xylan, wheat bran containing xylan, pulp, bagasse, corn fiber, rice straw or the like as a carbon source of liquid culture media.

In this technique, however, the microorganism to be cultured is a microorganism classified under the genus Bacillus. Further, an enzyme to be produced is xylanase, and the formation of β-glucanase has not been identified.

In other words, it has not been known to utilize wheat bran for a medium to simultaneously and highly produce β-glucanase and xylanase using a microorganism classified under the genus Trichoderma.

[Patent Literature 1] Japanese Patent Laid-open Publication No. 2003-137901
[Patent Literature 2] Japanese Examined Patent Application Publication No. H5(1993)-33984
[Patent Literature 3] Japanese Patent Laid-open Publication No. H9(1997)-163980
[Patent Literature 4] Japanese Patent Laid-open Publication No. H11(1999)-113568
[Patent Literature 5] Japanese Patent Laid-open Publication No. 2004-121257

### DISCLOSURE OF THE INVENTION

### PROBLEMS TO BE SOLVED BY THE INVENTION

The present invention solves the above conventional problems, and an object thereof is to produce cellulase which has excellent ability to decompose cellulosic resources containing xylan at low cost.

### MEANS FOR SOLVING THE PROBLEMS

As a result of intensive studies for a method for simultaneously and highly producing β-glucanase and xylanase, which decompose (glycosylate) cellulosic materials, and production of the enzymes, the present inventors found a method for simultaneously and highly producing β-glucanase and xylanase by culturing a microorganism classified under the genus Trichoderma by using (a) wheat bran and (b) ammonia nitrogen or amino nitrogen as materials of a liquid culture medium, and the liquid culture medium useful for producing the enzymes.

The present invention provides a method for producing β-glucanase and xylanase, comprising the step of culturing a microorganism classified under the genus Trichoderma by using a liquid culture medium which contains (a) wheat bran as a carbon source and (b) ammonia nitrogen or amino nitrogen as a nitrogen source.

In one embodiment, the concentration of the wheat bran in the liquid culture medium is not less than 3% W/V.

In one embodiment, the concentration of the wheat bran in the liquid culture medium is from 4 to 15% W/V.

In one embodiment, the concentration of the ammonia nitrogen or amino nitrogen in the liquid culture medium is from 30 to 660 mM.

In one embodiment, the concentration of the ammonia nitrogen or amino nitrogen in the liquid culture medium is from 40 to 580 mM.

In one embodiment, the microorganism classified under the genus Trichoderma is Trichoderma reesei.

In one embodiment, the carbon source is added to the liquid culture medium in the course of culture.

In addition, the present invention provides a liquid culture medium comprising (a) wheat bran as a carbon source and (b) ammonia nitrogen or amino nitrogen as a nitrogen source, wherein the liquid culture medium is used for culturing a microorganism classified under the genus Trichoderma.

In one embodiment, the wheat bran is contained in the concentration of not less than 2% W/V.

In one embodiment, the ammonia nitrogen or amino nitrogen is contained in the concentration from 30 to 660 mM.

In addition, the present invention provides β-glucanase and xylanase produced by any of the above described methods.

In addition, the present invention provides a method for decomposing or glycosylating a cellulosic resource, characterized by using the β-glucanase and xylanase.

### EFFECTS OF THE INVENTION

The present invention contributes to solving environmental issues since it decreases food industrial waste by effectively using wheat bran. In addition, since β-glucanase and xylanase, cellulolytic enzymes, are simultaneously and highly produced, the present invention is extremely useful for glycosylation of natural cellulosic resources such as bagasse, rice straw and the like. Specifically, it is useful for biomass ethanol production which produces ethanol from cellulosic resources.

### BRIEF DESCRIPTION OF DRAWINGS

[Fig. 1] A graph showing changes in enzyme activity of culture supernatant fluids against concentrations of ammonium sulfate in 5% wheat bran culture media.
[Fig. 2] A graph showing changes in enzyme activity of culture supernatant fluids against concentrations of ammonium chloride in 5% wheat bran culture media.
[Fig. 3] A graph showing changes in enzyme activity of culture supernatant fluids against concentrations of diammonium phosphate in 5% wheat bran culture media.
[Fig. 4] A graph showing changes in enzyme activity of culture supernatant fluids against concentrations of leucine in 5% wheat bran culture media.
[Fig. 5] A graph showing changes in enzyme activity of culture supernatant fluids against concentrations of urea in 5% wheat bran culture media.
[Fig. 6] A graph showing changes in enzyme activity of culture supernatant fluids against concentrations of wheat bran in culture media of 480 mM ammonium sulfate.
[Fig. 7] A graph showing changes in enzyme activity of culture supernatant fluids against concentrations of ammonium sulfate in 1% crystalline cellulose culture media.
[Fig. 8] A graph showing changes in enzyme activity of culture supernatant fluids against concentrations of crystalline cellulose in culture media of 160 mM ammonium sulfate.
[Fig. 9] A graph showing changes in enzyme activity of culture supernatant fluids against concentrations of ammonium sulfate in 1% wheat bran culture media.
[Fig. 10] A graph showing changes in enzyme activity of culture supernatant fluids against concentrations of ammonium sulfate in 5% wheat bran culture media.
[Fig. 11] A graph comparing concentrations of glucose produced by glycosylating beer draff using each supernatant fluid of a 5% wheat bran culture medium obtained in Example 1 and a 1% crystalline cellulose culture medium obtained in Reference Example 2.
[Fig. 12] A graph comparing concentrations of glucose produced by glycosylating rice straw using each supernatant fluid of a 5% wheat bran culture medium obtained in Example 1 and a 1% crystalline cellulose culture medium obtained in Reference Example 2.

### BEST EMBODIMENT FOR CARRYING OUT THE INVENTION

### Liquid Culture Medium

The liquid culture medium of the present invention contains nutrients to grow microorganisms producing cellulolytic enzymes such as genus Trichoderma, genus Aspergillus, genus Acremonium, genus Sporotrichum, genus Penicillium, genus Talaromyces, genus Humicola, genus Neocallimastix, genus Thermomyces or genus Clostridium, and genus Streptomyces.

The liquid culture medium is prepared based on a liquid culture medium obtained by dissolving and suspending the following culture medium composition into 100 ml of water (generally called a Mandel medium), and generally contains water as a medium, wheat bran as a carbon source and ammonia nitrogen or amino nitrogen as a nitrogen source. One example of preferred medium compositions is shown below.

### Medium composition:

Containing wheat bran: 5 g, (NH₄)₂SO₄: 0.14 g, KH₂PO₄: 1.5 g, CaCl₂•2H₂O: 0.03g, MgSO₄•7H₂O: 0.03 g, corn steep liquor: 2 mL, Tween 80: 0.1 mL, trace element solution (a solution of H₃ BO₄ 6 mg, (NH₄)₆Mo₇O₂₄•4H₂O 26 mg, FeCl₃•6H₂O 100 mg, CuSO₄•5H₂O 40 mg, MnCl₂•4H₂O 8 mg, ZnSO₄•7H₂O 200 mg): 0.1 mL, and water: 100 mL (adjusted to pH 4.8 using phosphoric acid or sodium hydroxide)

Wheat bran refers to a composite of wheat husks and germs. Wheat flour is obtained by removing wheat bran (i.e. husks and germs) from wheat and pulverizing the rest. Wheat bran is easily available because, for example, it is generated in large amounts as by-products in the milling step for industrially obtaining edible flour. Because the wheat bran is by-products of food production as described above, quality investigations and production process control are strictly performed at the stage of materials. Thus, the wheat bran is excellent in the hygiene quality and is safe and preferably used for the method of the present invention.

The type of wheat to prepare wheat bran is not particularly limited, and can include Hokushin, Fukusayaka, Norin 61, Nanbukomugi, Kitanokaori, Haruyutaka, Haru yo Koi and the like.

The wheat bran particles are generally in the shape of flakes. Wheat bran in the shape of flakes can be used as it is. It can also be used after suitably pulverized into fine particles and after forming particle masses by granulation. The forms of wheat bran are, for example, large bran, small bran, flour middlings and the like. Commercially available products such as food materials, health food and the like can also be used.

Wheat bran can be pretreated when introduced into the liquid culture medium. Preferred pretreatment is, for example, pulverization treatment and delignification treatment. When lignin is removed from wheat bran, solid cell walls are destroyed, and cellulose can be easily utilized. Thus, enzymes are easily produced. Also, the delignification treatment can be more efficiently performed by pulverization treatment of wheat bran.

Methods of delignification treatment are not particularly limited, and examples thereof include a method for decomposing by heating at high temperature in the presence of a strongly alkaline substance such as sodium hydroxide or in the presence of a strongly acidic substance such as sulfuric acid or phosphoric acid; a method for decomposing by microorganisms; and a method for decomposing by hydrothermal treatment under high temperature and high pressure. The method for decomposing by hydrothermal treatment under high temperature and high pressure is preferred in terms of a burden on treatment equipment and environment.

Pretreatment which is usually performed on materials for liquid culture media such as heat sterilization and the like can be further performed.

The concentration of the wheat bran in the liquid culture medium is preferably not less than 3% W/V. When the concentration of the wheat bran is less than 3% W/V, the production amount of cellulase, especially β-glucanase, may not increase so much. The concentration of the wheat bran in the liquid culture medium is more preferably not less than 4% W/V, and further preferably not less than 5% W/V, not less than 6% W/V, not less than 7% W/V and not less than 8% W/V.

The higher the concentration of the wheat bran in the liquid culture medium, the better. In other words, the upper limit is a limit amount at which the liquid culture medium can be stirred and mixed. When the liquid culture medium cannot be stirred, microorganisms are not uniformly mixed in the liquid culture medium, thus culture does not normally proceed.
The upper limit of the concentration of the wheat bran in the liquid culture medium can be 20, 15, 10 or 8% W/V in accordance with the performance of stirring equipment. Generally, the preferred range of the above concentration is from 4 to 15% W/V and preferably from 5 to 10% W/V.

The ammonia nitrogen refers to nitrogen contained in ammonia or ammonium salts derived from ammonia. The amino nitrogen refers to nitrogen contained in amines or amino compounds derived from amines. Examples of compounds which contain ammonia nitrogen or amino nitrogen include ammonium sulfate, ammonium nitrate, diammonium phosphate, ammonium chloride, aqueous ammonia, urea, and amino acids and salts thereof (e.g. leucine and sodium glutamate).

Among these, a particularly preferred compound to be used as a nitrogen source in the liquid culture medium of the present invention is ammonium sulfate. The reason is that it is low cost and easily available.

The concentration of the ammonia nitrogen or amino nitrogen in the liquid culture medium is from 30 to 660 mM as the number of moles of ammonium. Preferably, the concentration is from 40 to 580 mM. When the concentration is less than 30 mM, the production amount of cellulase, especially β-glucanase, may not increase so much. When the concentration exceeds 660 mM, productivity of the enzymes decreases. It is preferred that the concentration of the ammonia nitrogen or amino nitrogen in the liquid culture medium is increased and decreased in accordance with the concentration of the wheat bran in the liquid culture medium. For example, when the concentration of the wheat bran is 3% W/V, preferred is 50 mM in terms of cost and the like.

### Method for Producing β-Glucanase and Xylanase

The microorganisms classified under the genus Trichoderma used in the present invention are not particularly limited as long as they can produce cellulase which is required for glycosylation of cellulose. A preferred microorganism classified under the genus Trichoderma is a filamentous fungus of the genus Trichoderma, specifically Trichoderma reesei or Trichoderma viride. Trichoderma reesei is particularly preferred in terms of simultaneously and highly producing β-glucanase and xylanase.

Mycological properties of Trichoderma reesei and Trichoderma viride are described in, for example, E.G. Simmons, Abst. 2nd International Mycological Congress, Tampa, Florida, U.S., August 1977, page 618.

Using a conventional aeration-agitation culture device, liquid culture is performed at a culture temperature of 20 to 33°C, and preferably 28 to 30°C, at a culture pH of 4 to 6 for 4 to 10 days using the above liquid culture medium. When the above liquid culture medium is used from the beginning of culture, concentrations of ingredients (e.g. a carbon source and a nitrogen source) contained in the liquid culture medium correspond to the initial concentrations of the above ingredients in the culturing method of the present invention.

A carbon source may be added to the liquid culture medium in the course of culture. The reason is that the production efficiency of cellulase may improve by supplementing the carbon source since the wheat bran in the culture medium is decomposed with the progression of culture. As the carbon source to be added, a material which contains natural cellulose and is low in nutrients such as starchiness and the like is preferred. Preferred carbon sources to be added are wheat bran, beer draff, barley tea grounds, paper, squeezed residue of fruits (specifically squeezed residue of apples) and the like.

When a carbon source is added, the adding method may be a continuous or batch system, and the adding time and amount may be adjusted in order that media can be stirred and mixed after addition.

When a carbon source is added, ammonia nitrogen or amino nitrogen may be appropriately added in the course of culture as necessary.

Subsequently, if necessary, fungus bodies are removed from the culture liquid by known methods such as centrifugation, filtration and the like to obtain a culture supernatant fluid of a filamentous fungus of the genus Trichoderma. The culture liquid or culture supernatant fluid of the filamentous fungus of the genus Trichoderma contains the intended cellulase, i.e. β-glucanase and xylanase, in high concentration.

β-Glucanase activity of the culture liquid or culture supernatant fluid to be obtained is not less than 30 U/mL, preferably not less than 50 U/mL, more preferably not less than 60 U/mL and further preferably not less than 70 U/mL. Also, xylanase activity of the culture liquid or culture supernatant fluid is not less than 25 U/mL, preferably not less than 30 U/mL, more preferably not less than 40 U/mL, and further preferably not less than 50 U/mL. When either β-glucanase activity or xylanase activity of the culture liquid or culture supernatant fluid falls below the above lower limit, effects on the purpose of effectively utilizing a variety of naturally-occurring cellulosic resources decrease.

The above hemicellulase activity can be quantified based on an increase in absorbance at 540 nm by reacting reducing sugars produced by enzymatic hydrolysis of xylan derived from "oat spelts" as a substrate with DNS.

More specifically, to 1.9 mL of a 1% xylan substrate solution ("Xylan, from oat spelts" manufactured by Sigma is dissolved in a 200 mM acetic acid buffer solution (pH 4.5)), 0.1 mL of the culture liquid or culture supernatant fluid is added, and the obtained solution is enzymatically reacted at 40°C for exactly 10 minutes. Then, 4 mL of a DNS reagent (containing 0.75% dinitrosalicylic acid, 1.2% sodium hydroxide, 22.5% potassium sodium tartrate tetrahydrate, and 0.3% lactose monohydrate) is added thereto, and the obtained solution is mixed well to stop the reaction. In order to quantify the amount of reducing sugars contained in the reaction stop solution, the reaction stop solution is heated in a boiling-water bath for exactly 15 minutes. Next, the reaction stop solution is cooled to room temperature, and the amount of reducing sugars corresponding to xylose is then quantified by determining absorbance at 540 nm. One unit of the hemicellulase activity is represented as the enzyme amount which produces the reducing sugars corresponding to 1 µmol of xylose in 1 minute under the reaction conditions of 40°C and 10 minutes.

As used herein, "culturing a microorganism classified under the genus Trichoderma" refers to an operation of growing the microorganism according to common techniques. That is, in a method of performing liquid culture for the purpose of producing β-glucanase and xylanase, if at least a process, wherein a microorganism classified under the genus Trichoderma grows in the above liquid culture medium of the present invention, exists, the culturing method corresponds to the method of the present invention.

When culture is performed, nutrients in the liquid culture medium decrease since a microorganism classified under the genus Trichoderma consumes them. Thus, the concentrations of a carbon source and a nitrogen source in the culture medium at the end of culture are under the given concentrations. Consequently, a microorganism classified under the genus Trichoderma may grow in a culture medium, which does not correspond to the liquid culture medium of the present invention. Even in the above situation, when a liquid culture medium to be used corresponds to the liquid culture medium of the present invention containing a carbon source and a nitrogen source at given concentrations at, for example, the initiation of culture, the microorganism classified under the genus Trichoderma grow in the liquid culture medium of the present invention at least at the beginning of culture. Therefore, the culturing method clearly corresponds to the method of the present invention.

When a carbon source is contained in a large amount, particularly, at the beginning of culture, as described above, it is preferred that the upper limit of the concentration of wheat bran be limited to a certain level in terms of convenience when stirring and mixing the liquid culture medium.

On the contrary, even if the concentration of a carbon source or a nitrogen source in the culture medium at the beginning of culture is lower than a given one, and culturing is performed using a culture medium which does not correspond to the liquid culture medium of the present invention, when, for example, the concentrations of the carbon source and the nitrogen source in the culture medium become higher than the given concentrations by later adding the sources, since a microorganism classified under the genus Trichoderma grows in the liquid culture medium of the present invention, the culturing method corresponds to the method of the present invention.

### Method for Decomposing or Glycosylating Cellulosic Resources

The β-glucanase and xylanase obtained by the method of the present invention are useful for decomposing or glycosylating cellulosic resources. As used herein, cellulosic resources may be either synthetic cellulose or natural cellulosic resources. The synthetic cellulose represents cellulose distributed as cellulose powder. The natural cellulosic resources include bagasse, rice straw, wheat straw, beer draff, wood, and the like. The present invention can simultaneously and highly produce β-glucanase and xylanase, thus it excels in glycosylating natural cellulosic resources, specifically, bagasse, rice straw, wheat straw, beer draff and the like.

The method for decomposing or glycosylating cellulosic resources can be performed using known methods, and is not particularly limited. One example includes a method in which a cellulose resource is suspended in an aqueous medium as a substrate, and then the above culture liquid or culture supernatant fluid is added thereto, followed by performing glycosylation reaction by heating while stirring or shaking. In place of the above described culture liquid or culture supernatant fluid which shows cellulolytic activity, dry matters thereof or solutions obtained by dispersing or dissolving the dry matters in water may also be used.

It is preferred that cellulosic materials be preliminarily delignified. The reaction conditions such as a suspending method, a stirring method, a method for adding the above mixed solution, the order of addition, concentrations thereof and the like are appropriately adjusted to obtain glucose in higher yield.

The pH and temperature of the reaction solution may be within the range in which enzymes are not deactivated, and generally, when the reaction is carried out under normal pressure, the temperature and pH may be in the range of 30 to 70°C and of 3 to 7, respectively. In addition, the pressure, temperature and pH are appropriately adjusted to obtain glucose in higher yield as described above, and it is preferred that the reaction be carried out in an acetic acid- or phosphate-buffer solution under normal pressure at a temperature of 50 to 60°C and a pH of 4 to 6. The reaction time is generally from 6 to 147 hours, and preferably from 24 to 72 hours.

An aqueous solution containing glucose is obtained by glycosylation of cellulose. The obtained aqueous solution can be subjected to purification treatment such as decolorization, desalination, enzyme removal and the like as necessary. The purification method is not particularly limited as long as it is a known method. For example, activated carbon treatment, ionexchange resin treatment, chromatography treatment, filtration treatments such as microfiltration, ultrafiltration, reverse osmosis filtration and the like, crystallization treatment and the like may be used. These may be used alone or two or more may be used in combination.

The aqueous solution mainly composed of glucose purified by the above method can be used as it is, and may be solidified by drying as necessary. The drying method is not particularly limited as long as it is a known method. For example, spray drying, freeze drying, drum drying, thin-film drying, tray drying, flash drying, vacuum drying and the like may be used. These may be used alone or two or more may be used in combination.

### EXAMPLES

The present invention will now be described in more detail by way of examples, but the present invention is not limited thereto.

### Example 1

Wheat bran (manufactured by Showa Sangyo Co., Ltd.) was pulverized, and lignin was removed by autoclave treatment in a 0.3 N aqueous solution of sodium hydroxide at 121°C for 15 minutes. The resultant was sufficiently washed with water, followed by drying.

Trichoderma reesei QM9414 (NBRC 31329) was cultured on a potato dextrose agar medium at 28°C for 7 days to sufficiently form spores. In a Mandel medium, crystalline cellulose, a carbon source, was replaced with 5% delignified wheat bran (5 g/100 mL), and ammonium sulfate, a nitrogen source, was added thereto so that the molar concentration of amino nitrogen would be each 15 mM, 35 mM, 50 mM, 65 mM, 80 mM, 100 mM or 115 mM. The obtained solutions were adjusted to pH 4.8 using phosphoric acid or sodium hydroxide, and 100 mM of liquid culture media were prepared in 500 mL baffled Erlenmeyer flasks. A loopful of the cultured Trichoderma reesei was inoculated into the liquid culture media, and they were cultured with shaking at 28°C, 180 rpm for 7 days. The culture liquids were centrifuged on day 7, and β-glucanase activity and xylanase activity of the supernatant fluids were determined.

### (Determination of Enzyme Activity)

Enzyme activity of the culture liquids obtained above was determined.
For β-glucanase activity, absorbance of a dyed fragment generated by enzymatic decomposition of dye-labeled β-glucan as a substrate was determined using a β-Glucanase Assay Kit manufactured by Megazyme. Specifically, 0.1 mL of the culture liquid was added to 0.1 mL of an azo-barley glucan substrate solution, and the obtained solution was enzymatically reacted at 40°C for exactly 10 minutes. Then, 0.6 mL of a stop solution [containing 4% sodium acetate, 0.4% zinc acetate and 80% methyl cellosolve (pH 5)] was added thereto, followed by leaving to stand the obtained solution for 5 minutes to stop the reaction. Subsequently, the obtained solution was centrifuged, and absorbance of the supernatant fluid at 590 nm was determined. One unit of β-glucanase activity was represented as the enzyme amount which produces reducing sugars corresponding to 1 µmol of glucose in 1 minute under the reaction conditions of 40°C and 10 minutes.

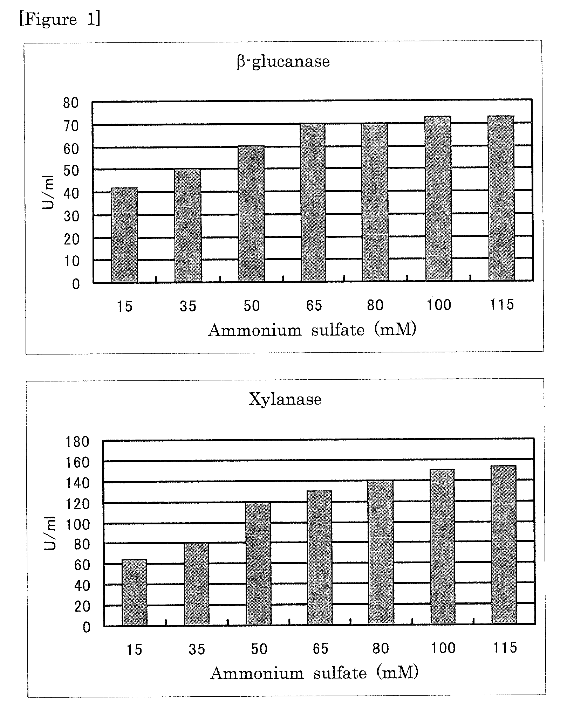

Next, xylanase activity was quantified as an increase in absorbance at 540 nm by reacting reducing sugars produced by enzymatic hydrolysis of xylan derived from "oat spelts" as a substrate with DNS. More specifically, to 1.9 mL of a 1% xylan substrate solution [Xylan, from oat spelts, manufactured by Sigma was dissolved into a 200 mM acetic acid buffer solution (pH 4.5)], 0.1 mL of the culture liquid was added, and the obtained solution was enzymatically reacted at 40°C for exactly 10 minutes. Then, 4 mL of the DNS reagent (containing 0.75% dinitrosalicylic acid, 1.2% sodium hydroxide, 22.5% potassium sodium tartrate tetrahydrate and 0.3% lactose monohydrate) was added thereto, and the obtained solution was mixed well to stop the reaction. In order to quantify the amount of reducing sugars contained in the reaction stop solution, the reaction stop solution was heated in a boiling-water bath for exactly 15 minutes. Next, the reaction stop solution was cooled to room temperature, and the amount of reducing sugars corresponding to xylose was quantified by determining absorbance at 540 nm. One unit of xylanase activity was represented as the enzyme amount which produces reducing sugars corresponding to 1 µmol of xylose in 1 minute under the reaction conditions of 40°C and 10 minutes. The results are shown in Fig. 1.

### Example 2

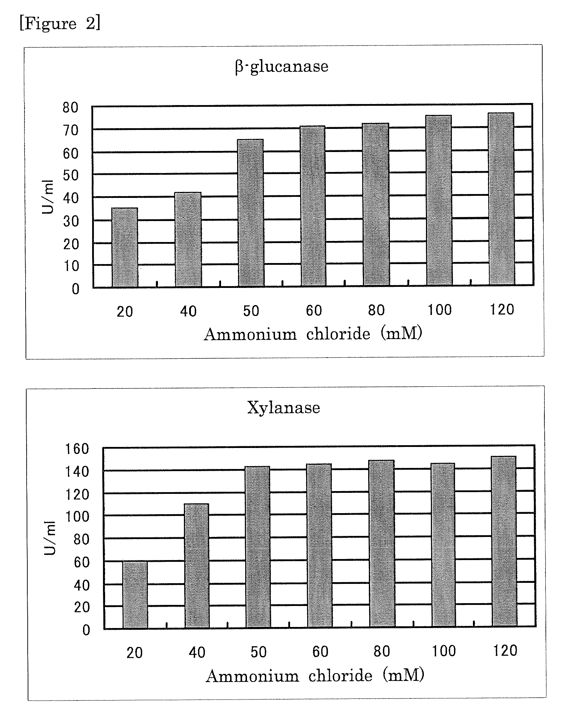

In a Mandel medium, crystalline cellulose, a carbon source, was replaced with 5% delignified wheat bran (5 g/100 mL) obtained in the same manner as in Example 1, and ammonium sulfate, a nitrogen source, was replaced with ammonium chloride, and the ammonia chloride was added thereto so that the molar concentration of ammonia nitrogen would be each 20 mM, 40 mM, 50 mM, 60 mM, 80 mM, 100 mM or 120 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 2.

### Example 3

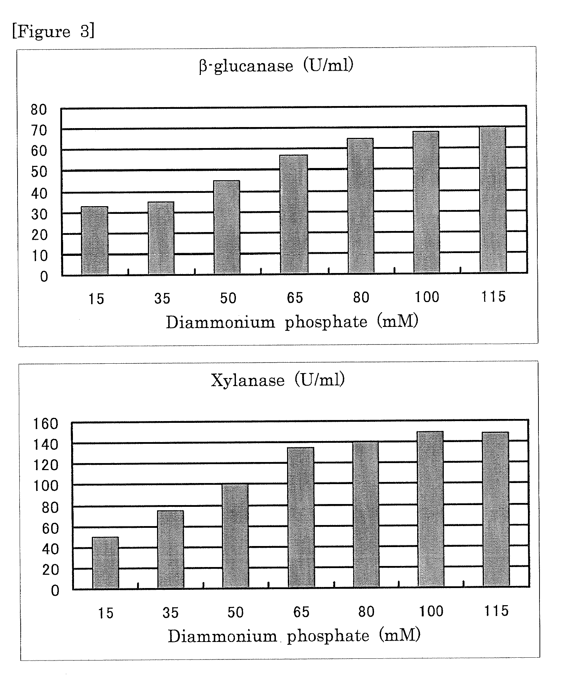

In a Mandel medium, crystalline cellulose, a carbon source, was replaced with 5% delignified wheat bran (5 g/100 mL) obtained in the same manner as in Example 1, and ammonium sulfate, a nitrogen source, was replaced with diammonium phosphate, and the diammonium phosphate was added thereto so that the molar concentration of ammonia nitrogen would be each 15 mM, 35 mM, 50 mM, 65 mM, 80 mM, 100 mM or 115 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media and they were cultured with shaking at 28°C, 180 rpm for 7 days. The culture liquids were centrifuged on day 7, and β-glucanase activity and xylanase activity were determined in the same manner as in Example 1. The results are shown in Fig. 3.

### Example 4

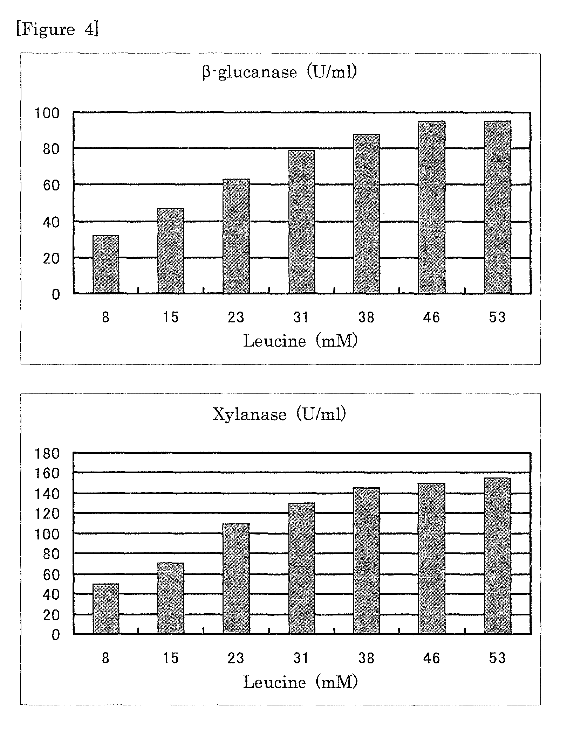

In a Mandel medium, crystalline cellulose, a carbon source, was replaced with 5% delignified wheat bran (5 g/100 mL) obtained in the same manner as in Example 1, and ammonium sulfate, a nitrogen source, was replaced with leucine, and the leucine was added thereto so that the molar concentration of amino nitrogen would be each 8 mM, 15 mM, 23 mM, 31 mM, 38 mM, 46 mM or 53 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 4.

### Example 5

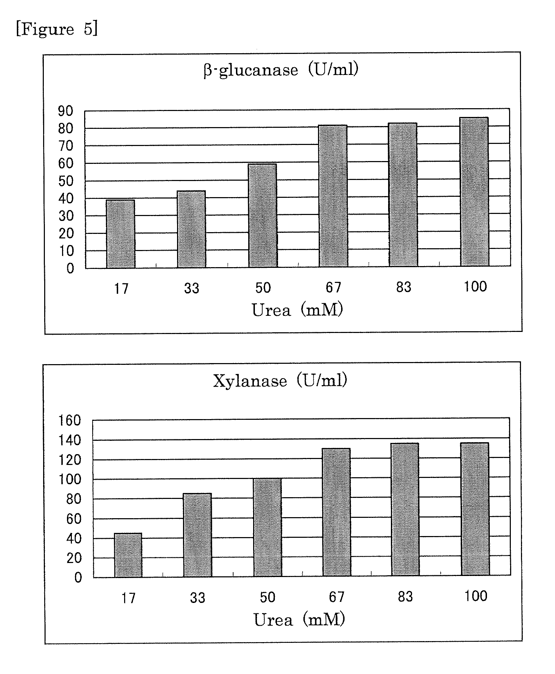

In a Mandel medium, crystalline cellulose, a carbon source, was replaced with 5% delignified wheat bran (5 g/100 mL) obtained in the same manner as in Example 1, and ammonium sulfate, a nitrogen source, was replaced with urea, and the urea was added thereto so that the molar concentration of amino nitrogen would be each 17 mM, 33 mM, 50 mM, 67 mM, 83 mM or 100 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 5.

### Example 6

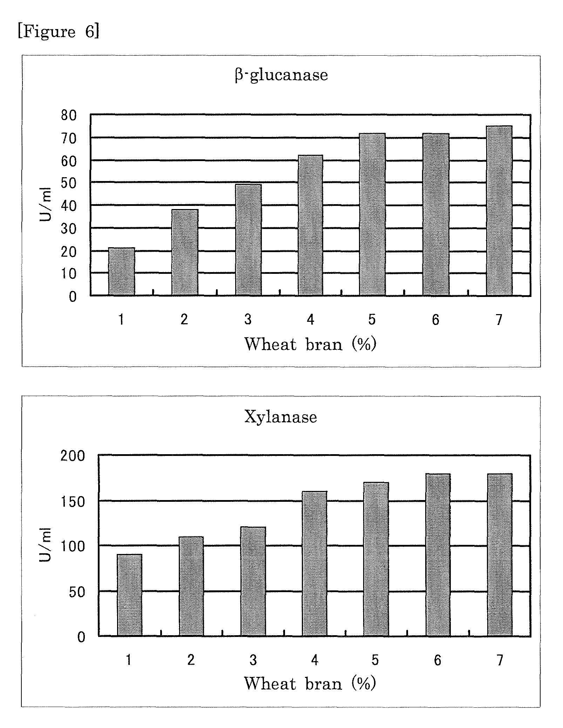

In a Mandel medium, crystalline cellulose, a carbon source, was replaced with the delignified wheat bran obtained in the same manner as in Example 1, and the delignified wheat bran was added thereto so that the concentration thereof would be 1%, 2%, 3%, 4%, 5%, 6% or 7%. Ammonium sulfate, a nitrogen source, was also added thereto so that the molar concentration of ammonia nitrogen would be 480 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 6.

### Reference Example 1

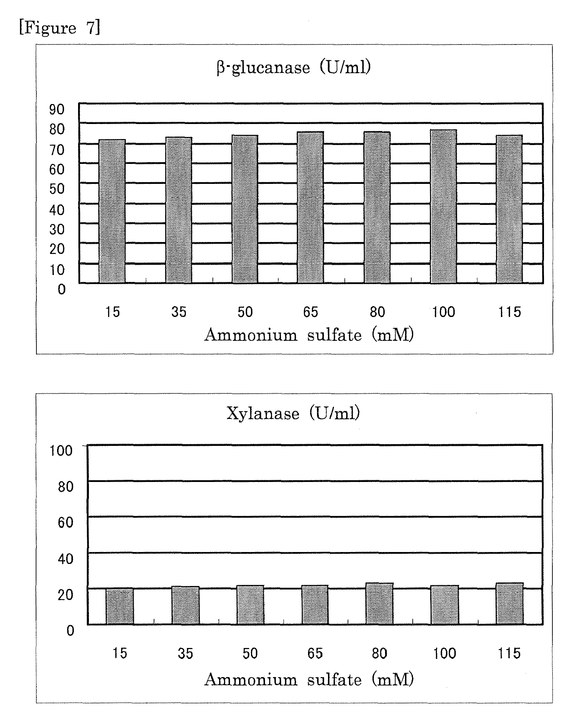

In a Mandel medium, the concentration of crystalline cellulose, a carbon source, was 1%, and ammonium sulfate, a nitrogen source, was added thereto so that the molar concentration of ammonia nitrogen would be each 15 mM, 35 mM, 50 mM, 65 mM, 80 mM, 100 mM or 115 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 7.

### Reference Example 2

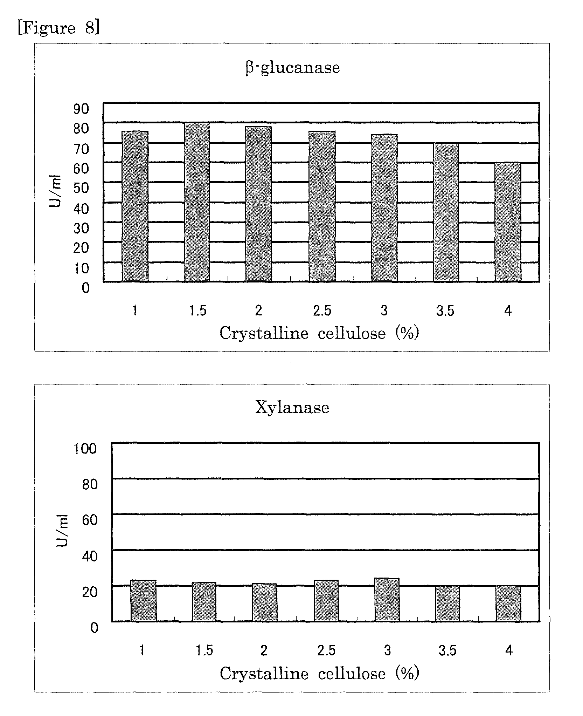

In a Mandel medium, crystalline cellulose, a carbon source, was added so that the concentration thereof would be 1%, 1.5%, 2%, 2.5%, 3%, 3.5% or 4%, and ammonium sulfate, a nitrogen source, was added thereto so that the molar concentration of ammonia nitrogen would be 160 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 8.

### Reference Example 3

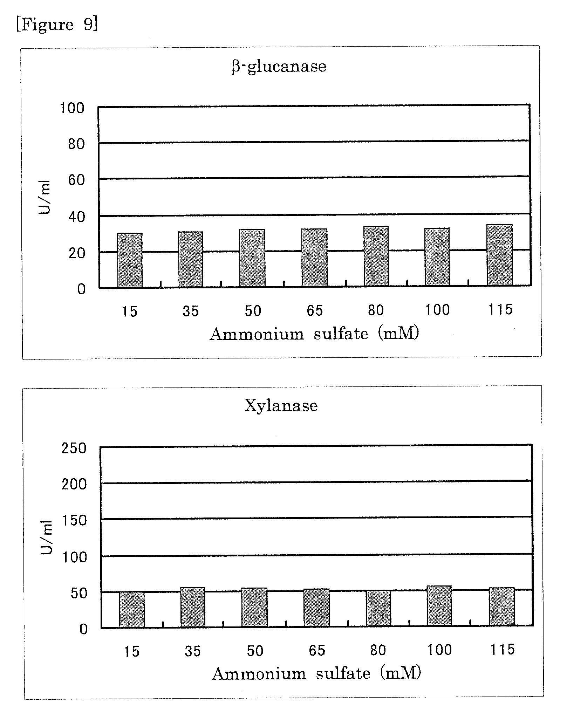

In a Mandel medium, crystalline cellulose, a carbon source, was replaced with 1% delignified wheat bran (1 g/100 mL) obtained in the same manner as in Example 1, and the delignified wheat bran was added thereto. Ammonium sulfate, a nitrogen source, was added thereto so that the molar concentration of ammonia nitrogen would be each 15 mM, 35 mM, 50 mM, 65 mM, 80 mM, 100 mM or 115 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 9.

### Example 7

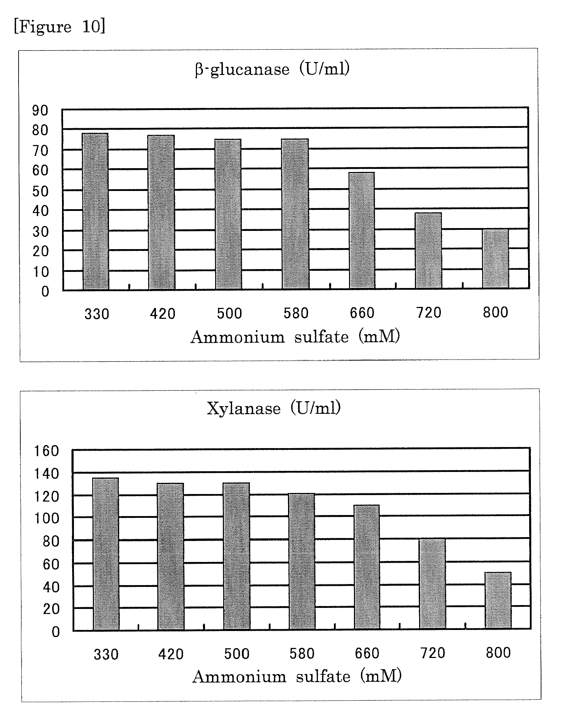

In a Mandel medium, crystalline cellulose, a carbon source, was replaced with 5% delignified wheat bran (5 g/100 mL) obtained in the same manner as in Example 1, and ammonium sulfate, a nitrogen source, was added thereto so that the molar concentration of ammonia nitrogen would be each 330 mM, 420 mM, 500 mM, 580 mM, 660 mM, 720 mM or 800 mM, and liquid culture media were prepared in the same manner as in Example 1. Trichoderma reesei QM9414 (NBRC 31329) was cultured on a potato dextrose agar medium at 28°C for 7 days to sufficiently form spores. A loopful of the spores was inoculated into the liquid culture media, and they were cultured with shaking at 28°C, 180 rpm for 7 days. The culture liquids were centrifuged on day 7, and β-glucanase activity and xylanase activity were determined in the same manner as in Example 1. The results are shown in Fig. 10.

### Example 8

A glycosylation test of cellulosic materials was performed using the culture supernatant fluid obtained in Example 1 (5% wheat bran and 100 mM ammonia nitrogen in ammonium sulfate) and the culture supernatant fluid obtained in Reference Example 2 (1% crystalline cellulose and 100 mM ammonia nitrogen in ammonium sulfate). Beer draff and rice straw were prepared as cellulosic materials to be used for glycosylation. These cellulosic materials were delignified by the following method.

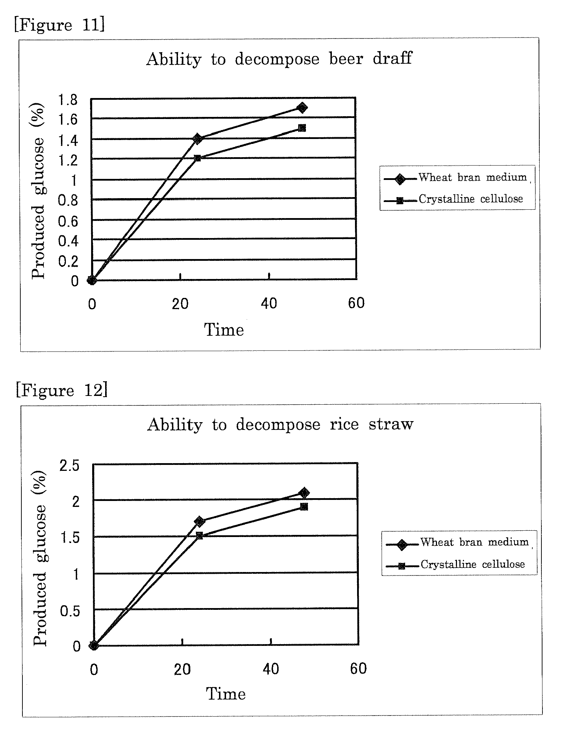

A cellulosic material was pulverized, and the obtained pulverized substance was suspended in 0.3 N NaOH. The suspension was treated at 120°C for 15 minutes, and the resultant was adequately washed with water, followed by drying. In glycosylation of the cellulosic material, a solution (a 3% solution of the cellulosic material), which was composed of the cellulosic material: 0.3 g, the culture supernatant fluid: 9.5 mL, and 1 M acetic acid buffer (pH 4.8): 0.2 mL, was shaken at 50°C, pH 4.8 for 48 hours to glycosylate, and the produced glucose was determined by Glucose CII-Test Wako (Wako Pure Chemical Industries, Ltd.). The results are shown in Figs. 11 and 12.

### INDUSTRIAL APPLICABILITY

β-Glucanase and xylanase, which are extremely useful for glycosylation of natural cellulosic resources such as bagasse, rice straw and the like, can be simultaneously and highly produced. The production method can be utilized for biomass ethanol production which produces ethanol from cellulosic resources.

## Claims

1. A method for producing β-glucanase and xylanase, comprising the step of culturing a microorganism classified under the genus Trichoderma by using a liquid culture medium which contains (a) wheat bran as a carbon source and (b) ammonia nitrogen or amino nitrogen as a nitrogen source.

2. The method for producing β-glucanase and xylanase according to claim 1, wherein the concentration of the wheat bran in the liquid culture medium is not less than 3% W/V.

3. The method for producing β-glucanase and xylanase according to claim 1 or 2, wherein the concentration of the wheat bran in the liquid culture medium is from 4 to 15% W/V.

4. The method for producing β-glucanase and xylanase according to any of claims 1 to 3, wherein the concentration of the ammonia nitrogen or amino nitrogen in the liquid culture medium is from 30 to 660 mM.

5. The method for producing β-glucanase and xylanase according to any of claims 1 to 4, wherein the concentration of the ammonia nitrogen or amino nitrogen in the liquid culture medium is from 40 to 580 mM.

6. The method for producing β-glucanase and xylanase according to any of claims 1 to 5, wherein the microorganism classified under the genus Trichoderma is Trichoderma reesei.

7. The method for producing β-glucanase and xylanase according to any of claims 1 to 6, wherein the carbon source is added to the liquid culture medium in the course of culture.

8. A liquid culture medium comprising (a) wheat bran as a carbon source and (b) ammonia nitrogen or amino nitrogen as a nitrogen source, wherein the liquid culture medium is used for culturing a microorganism classified under the genus Trichoderma.

9. The liquid culture medium according to claim 8, comprising not less than 2% W/V of the wheat bran.

10. The liquid culture medium according to claim 8 or 9, comprising from 30 to 660 mM of the ammonia nitrogen or amino nitrogen.

11. β-Glucanase and xylanase produced by the method according to any one of claims 1 to 7.

12. A method for decomposing or glycosylating a cellulosic resource, **characterized by** using the β-glucanase and xylanase according to claim 11.
